# EUROPEAN PATENT SPECIFICATION

(11) **EP 1 137 459 B1**
(45) Date of publication and mention of the grant of the patent: **24.01.2007**
(21) Application number: 99955650.9
(22) Date of filing: 24.11.1999
(51) Int. Cl.: A61N 5/073, A61H 23/02, F21V 9/14

(54) **INSTRUMENT FOR LIGHT THERAPY**
EINRICHTUNG ZUR LICHTTHERAPIE
APPAREIL POUR PHOTOTHERAPIE

(30) Priority: 24.11.1998 CZ 382398; 09.11.1999 CZ 1005899 U
(43) Date of publication of application: 04.10.2001
(73) Proprietor: Voves, Vladimir, 395 26 Lukavec (CZ)
(72) Inventor: VOVES, Vladimir, 395 26 Lukavec (CZ); PICKA, Pavel, 395 01 Pacov (CZ)
(74) Representative: Fischer, Michael
(86) International application number: PCT/CZ1999/000044
(87) International publication number: WO 2000/030714

(56) References cited:
- EP-A- 0 137 005
- WO-A-96/04958
- WO-A-96/04959
- DE-A- 3 220 218
- DE-C- 3 733 905

## Description

The invention concerns an instrument for light therapy, consisting of an outer casing with a front section terminated with the first hole, in which inner casing with a light source, reflector and Brewster polariser consisting of a system parallel glass plates lying on each other are located, while the output section of the inner casing reaching as far as the first hole of the outer casing holds a light filter, and with a rear section that changes into the second hole, in which fan for generation of pressure difference between both holes is located.

Instruments for light therapy are used to support biological processes by means of effects of linear-polarised light. Action of the linear-polarised light increases cell activity and supports healing processes of various disturbances of body surface such as wounds, furuncles and various disturbances of epithelium. In the publication DE 3220218 an equipment and a method are described for a stimulation of biological processes and for activating cells by means of linear-polarised light. The Brewster polariser consists of a larger number of plane-parallel glass plates from common transparent glass inclined under the known Brewster angle, e.g. four glass plates with a double number of reflection planes that reflect about 35% of incident light. The Brewster polariser is placed in a cylindrical cabinet with the same diameter as the reflector and lens body. The cylindrical cabinet is cut in the axis with an inclined plane and glass plates are inserted into the ellipse-shaped cross-section. Rays pass through under an obtuse angle that corresponds to the double of the Brewster angle. The linear-polarised light has continuous or seemingly continuous distribution of spectrum in the usable direction. Despite the indisputable healing effects, the bundle of rays generated by the light source generates too much heat at higher power output that in case of the current configuration cannot be satisfactorily removed by means of air from fan. Another disadvantage of this appliance is a complicated construction of the optical equipment. The publication WO 96/04958 describes a healing lamp with a Brewster polariser formed by several drawn glass plates lying directly one on the other. These glass plates provide better heat removal thanks to a close contact of glass plates and thanks to cooling air passing through a narrow gap between the casing jacket and system of glass plates of the Brewster polariser. Because the light space with reflector, light source, glass plates of the Brewster polariser and output filter is separated from the space, through which air passes, and because flowing air has no access between glass plates of the Brewster polariser, the polariser is not polluted with dust. The cooling air passes through the casing of the instrument and leaves the casing in a different direction than to the cured area. The disadvantage of this configuration is a complicated manufacture of the whole instrument, low efficiency of light reflection and impossibility to use the airflow to support the instrument's healing effect. The publication DE 3733905 describes a healing lamp with linear-polarised light. With this lamp, the reflector with a light source generates a bundle of rays that impinges on the Brewster polariser made of a larger number of plane-parallel glass plates configured next to each other at small determined distances. Glass plates are enclosed in a metal plate, whose outer surface is fitted with cooling fins as well as the outer surface of the reflector. The healing lamp consists of two tubular casings that make an angle that corresponds to the Brewster angle. The Brewster polariser is located in the cranked section. The straight section is tightly closed by means of a filtration insert. The disadvantage of the above-described healing lamp is its robust construction necessary to achieve a satisfactory light performance. Documentation EP 0137005 describes a healing illumination instrument consisting of a crank-shaped cylindrical casing, whose cranked section accommodates the Brewster polariser plates. One of the straight sections of the casing holds a light source with reflector, which transmits a bundle of rays onto the Brewster polariser plates. The bundle of rays reflects from these plates into the second straight section of the casing and the light beam leaves the instrument at the output hole of the second straight section. This hole is arranged around an optical appliance, which can be, for example, an optical filter. A fan is located after the reflector in the first straight section of the casing. This fan causes air to be sucked from the output hole area into the second straight section, the air passes through the cranked section of the cylindrical casing and a peripheral gap along the reflector into the fan that forces the air out from the casing through holes in the facing wall of the first straight section. The fan can be set to the opposite direction of airflow, in which case the air is sucked in through holes in the facing wall of the first straight section, passes through the fan and is pushed through an annular gap between the reflector and casing. Then the air flows along the top plate of the Brewster polariser and after changing the direction in the cranked section of the casing, the air flows through the second straight section through annular holes along the optical equipment out of the instrument. In case of the latter direction of flow, the air flows to the healed area and around. However, disadvantage of this instrument is that in case of this direction, the air flows directly along the top plate of the Brewster polariser and hits the optical equipment located in the output hole of the second section and pollutes it with dust. Documentation WO 96/04959 describes a healing lamp for generation of linear-polarised light that consists of an outer casing with a shape of cranked cylinder with holes on both sides, and of an inner casing located inside the outer casing. The inner casing also has a shape of a cranked cylinder. One end of the inner casing is fitted with a reflector with a light source transmitting a bundle of rays onto plates of the Brewster polariser located at the area of cranking of the cylindrical shape of the inner casing. The bundle reflects from the plates of the Brewster polariser and leaves the instrument through the output hole in the output section of the inner casing that terminates at the area of the outer casing output hole. An annular gap is formed between the output section of the inner casing and the first input hole of the outer casing. The fan located in the area of the second output hole of the outer casing sucks air that passes through this gap into the inner area of the outer casing. The fan forces the sucked air out of the outer casing through the second output hole. The air flows in the direction from the healed area, through the annular gap between the output section of the inner casing and the first output hole of the outer casing along the reflector and further along the inner electrical wiring into the fan and from the fan through the hole in the outer casing into the environment, which seems to be disadvantageous, because the flowing air is not utilised to increase the healing effects. The sucked air simultaneously carries a mass of biological contamination from the healed area into the annular gap between the output section of the inner casing and the first input hole of the outer casing. This biological contamination is undesirably deposited on the inner wiring of the instrument. This effect is undesirable, because this is a biological contamination, which is a source of various infections. Disadvantage of this healing lamp according to the above description also is a limited light performance and a limited healing effect. The limited light performance and healing effect are caused by low efficiency of light reflection on the Brewster polariser. The Brewster polariser according to the above description consists of several layers of drawn glass plates arranged directly on top of each other. Purchase costs of floated glass plates are high. The reflection efficiency of bundle of rays on the above glass plates is low and therefore also the light performance of the bundle of rays, that flows out from the output hole of the output section of the inner casing onto the healed area, is low. The low reflection efficiency of the light bundle on the glass plates of the Brewster polariser is caused by quality of the surface of drawn glass plates, because during production at temperatures in the glass production equipment, the drawn glass plates are placed or transported on guide plates made of zinc. At high temperatures of plates, zinc penetrates into the surface of the processed plates. Even the slightest content of zinc in the surface layer causes aggravation of the reflection capacity. The same effect also appears during the production of glass plates in every other method of production, in which guide plates have to be used, that with the current state of technology are exclusively made to contain zinc. Finally, documentation CZ 8371 describes a Brewster polariser that consists of a system of at least two adjoining glass reflection plates, where at least one black spacing insert is adjoining to one of its sides. The system of glass plates is inserted into the hole in the casing and locked together with the black spacing insert. Use of one black spacing insert cannot always comply with various manufacturing tolerances of glass reflection plates and their thermal expansion during operation of the instrument. This also causes a play between the glass reflection plates, which requires other black spacing inserts of different thickness to be used. The aim of the invention is to eliminate the disadvantage of the current state of the art and to provide an instrument for light therapy that has a higher light performance at the output, lower power consumption, generates less heat, is more hygienic and does not clog with contamination, does not clog optical equipment located at the output with contamination, enables improvement of existing healing effects and expansion of functions with massage effects, features a simple construction, can be handled more easily and features the same or lower demands and costs during production and assembly.

Disadvantages of the state of the art are eliminated to a considerable extent by the invention as defined in claim 1. The present invention provides an instrument for light therapy consisting of an outer casing with a front section terminated with the first hole, in which inner casing with a light source, reflector and Brewster polariser consisting of a system parallel glass plates lying on each other are located, while the output section of the inner casing reaching as far as the first hole of the outer casing holds a light filter, and with a rear section that changes into the second hole, in which fan for generation of pressure difference between both holes is located according to the invention consisting in that the system of glass plates of Brewster polariser is created by drawn glass plates with surface irregularities defining cavities of unequal shape between plates.

According to advantageous embodiment of the present invention the outer side of the system of glass plates can be provided with insert with black surface that has profile patterns on the side of glass plates, massage ring can be allocated to the Brewster polariser, that is located in output section of inner casing and/or at the end of front part of outer casing. With advantage the massage ring can be provided with flexible massage elements, advantageously can be the massage ring provided with for generating a magnetic field outside the output hole of inner casing. Magnets can have with advantage a permanent or an electrical excitation. With advantage the massage ring can be a movable part of vibrator located at output section of inner casing and/or at the end of front section of outer casing. According to an advantageous execution there is fan allocated to Brewster polariser whereas this fan is set for airflow direction from the second hole of outer casing toward its first hole. With advantage there is lens allocated to Brewster polariser, which is located in output section of inner casing to focus rays reflected from he system of glass plates nto the focus located outside outer casing on the optical axis of lens.

The construction is advantageous for improvement of the degree of utilisation of the reflected bundle of rays. In this construction, the elliptic reflector section is located against the parabolic reflector section so that optical axes of both reflector sections merge into a single optical axis. The light source is located in the primary focus of the elliptic reflector section, whose secondary focus is basically identical with that of the parabolic reflector section and the size of the elliptic reflector section is considerably smaller than that of the parabolic reflector. This system of reflectors, in fact, avoids diffuse light. With respect to simplification of production and reduction of requirements on accuracy of production of glass reflection plates, it is advantageous to use a construction, in which the Brewster polariser consists of three glass reflection plates that as a result of various surface irregularities create unequal gaps between each other and are terminated at least with one black spacing insert and are jointly inserted into the hole in the casing and locked by means of the carrier together with the black spacing insert. To enable simple amplification or attenuation of irradiation intensity or to regulate the size of the illuminated area, it is an advantage that the lens consists of a spherical convergent lens, whose focus lies on the optical axis in front of the cabinet in the direction of the outgoing light. The construction is also advantageous for improvement and intensification of programmable healing, irradiation and massage effects. According to this construction, a massage head is detachably fitted to the holder end. At its front part, this head is provided with flexible massage elements and is manually and/or electrically controlled. Circling motion of the massage head with flexible elements over the skin surface improves blood circulation in the skin and together with exhaustion of warm air it considerably improves the healing irradiation effects and adds also massage effects. Electrical control of the massage head, e.g. with vibrations, is another improvement of the instrument's function that enhances it utilisation in medicine and cosmetics. The vibration intensity and cycle length of the selected program can be remote controlled and changed by means of program buttons and buttons for amplification and attenuation of effects. Holder supports, air deflector for guiding air supplied by fan to cool the light source and Brewster polarised located within the cabinet are advantageous for amplification of the cooling effect. In order to keep cleanliness of the sucked air on the airtight construction of the optical equipment for guiding, polarisation and filtration of light, it is an advantage that air access holes are covered with a replaceable air filter, which helps to prevent contamination of the treated body surface areas. Further, it is advantageous to use the construction, in which the light filter has a colour shade that enables filtration of undesired light spectrum components. For use at rehabilitation centres when healing post-fracture, operation and burn states, the handling of the instrument is simplified so that the electrical outfit for the radiated light is fitted with a controller of program of healing, irradiation and massage functions controlled by means of program buttons and buttons for amplification and attenuation of effects with light and/or acoustic indication. This enables treatments to be performed at outpatient clinics and at beds without any undesired side effects. This is achieved by means of construction of the cabinet, enhanced construction of the Brewster polariser including simplification of glass reflection plates of the massage head and by means of introduction of remote control featuring a simple operation. The black spacing insert has a profiled surface that performs a function of a spring and flexibly compensates effects of manufacturing tolerances of thickness and thermal expansion of the system of glass reflection plates so that their tight seating onto each other in the hole of the inner casing after assembly is permanently guaranteed. Simple designation of the product and manufacturer is facilitated by the fact that the surface of the black spacing insert holds a marking, e.g. a trademark that is visible when looking into the instrument for light therapy against the direction of the outgoing polarised light. Thanks to the higher efficiency, the instrument for light therapy according to the invention has a considerably higher light performance at the output and thus lower power consumption and generates less heat at the outer side of glass plates of the Brewster polariser, it is more hygienic and does not clog with contamination, does not contaminate optical equipment located at the output with dust, thanks to devices allocated to the Brewster polariser it enables improvement of existing healing effects and expansion of functions with mechanical, magnetic and optical massage effects, it features a simple construction, is easy to handle and features the same or lower demands and costs during production and assembly.

The invention is explained in details by means of drawings. Figure 1 shows a longitudinal sectional view of the instrument for light therapy according to the invention. Figure 2 shows a detail of a longitudinal sectional view of glass plates of the Brewster polariser. Figure 3 shows a view of output section of the inner casing. Figure 4 shows a longitudinal sectional view of the massage ring with flexible massage elements. Figure 5 shows a longitudinal sectional view of the massage ring with permanent magnets and electromagnets. Figure 6 shows a longitudinal sectional view of massage ring consisting of a vibrator. Figure 7 shows an example of light path of bundle of rays.

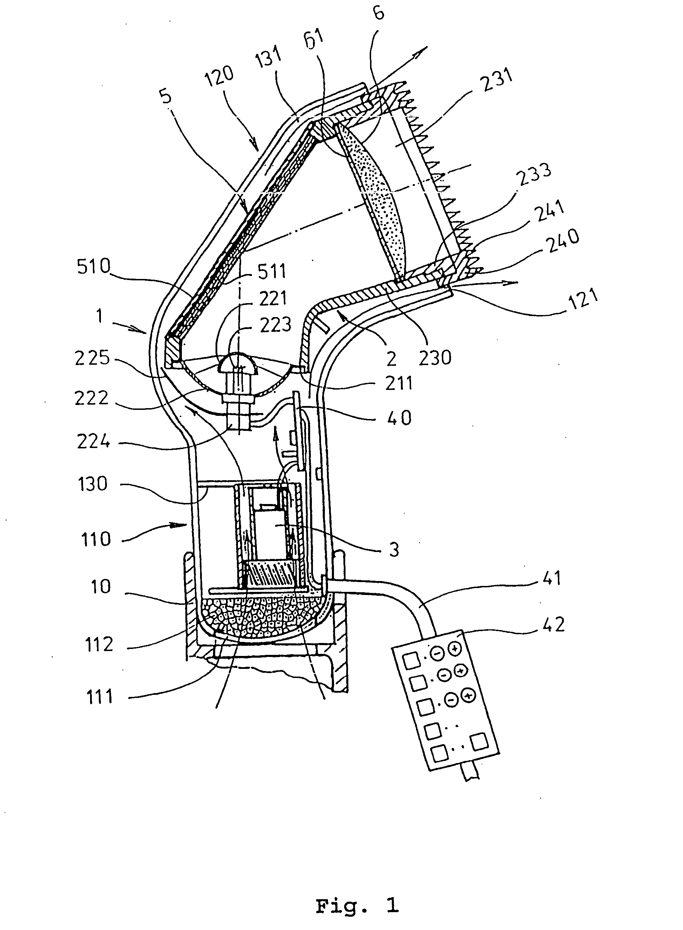

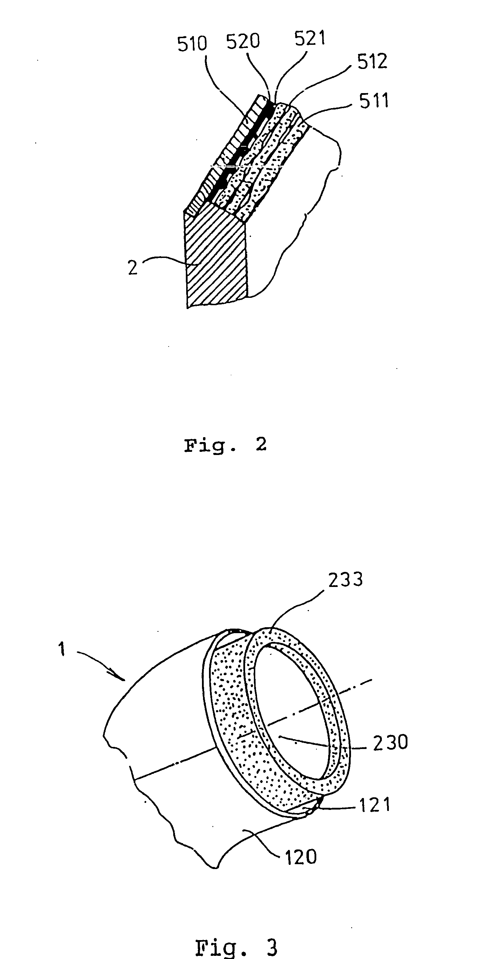

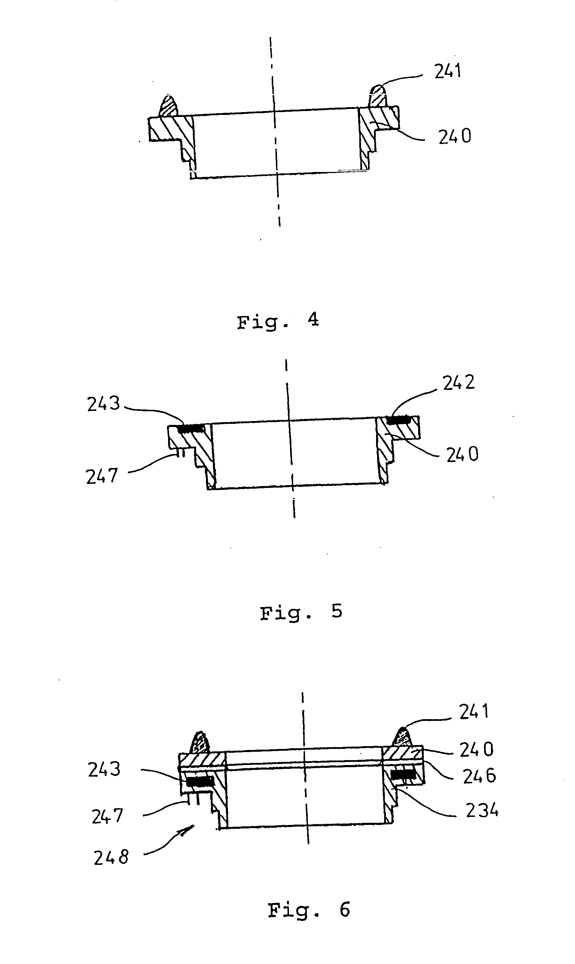

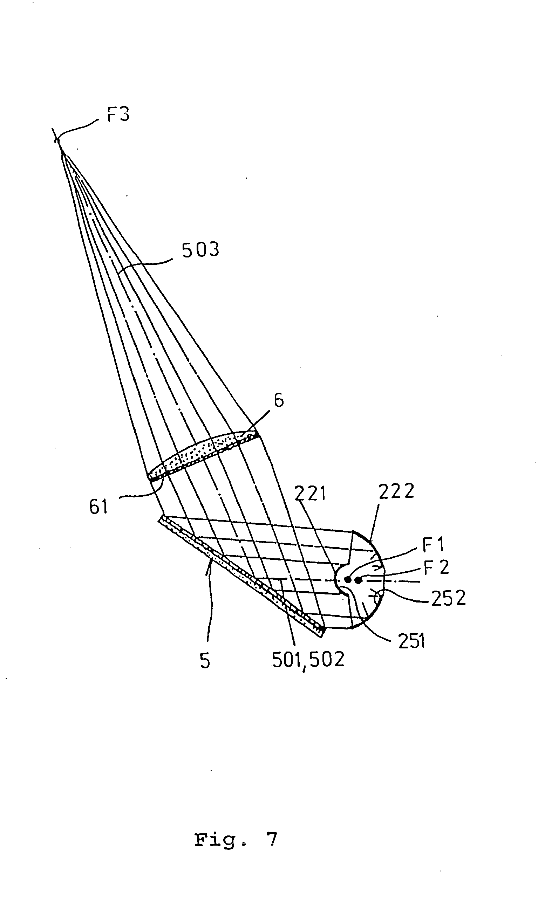

According to Fig. 1, the instrument for light therapy consists of an outer casing 1 made of plastic. It is an advantage, that this casing is divided into two sections longitudinally connected either with screws or by means of latches and clamps. In the longitudinal sectional view, the outer casing 1 is shown as a double contour line. Basically, the outer casing 1 has a shape of a cranked cylinder with front section 120 and rear section 110. Fins not shown in the figure hold the inner casing 2 in the front section 120. This casing also has the shape of cranked cylinder. The outer side of the cranked section holds Brewster polariser 5 consisting of a system of drawn glass plates 511 attached to bearing plate 510 fitted into inner casing 2. Inner casing 2 is provided with hole 211 at one of its end. Shoulder of the hole holds reflector 222 that focuses rays generated by light source 233 fitted within reflector 222 by means of socket 224 and partly covered by known method with the other reflector 221. The other end of inner casing 2 changes into output section 230 with output hole 231. The recess (not show in the figure) of output section 230 of inner casing 2 holds filter 61 and lens 6 is located outside filter 61. Filter 61 and lens 6 are fixed in output section 230 of inner casing 2 by means of annular ring 233 of a suitable shape. Annular ring 233 holds massage ring 240 fitted with massage elements 241. It is an advantage that massage elements 241 can be made of a flexible material so that contact with the treated area does not cause irritation, but a desirable massage. Front section 120 of outer casing 1 changes into the rear section 110 either gradually or in the form of a crank. This rear section 110 holds fan 3 fitted in the outer casing, for example, by means of internal ribs 130. Rear section 110 of outer casing 1 is terminated with hole 111 in front of which air filter 112 is fitted. Supply cable 41 leading to switchboard 40 is taken from controller 42 to rear section 110 of outer casing 1. Cables are distributed from here to socket 224 of light source 223 and to fan 3. With its rear section 110, the instrument for light therapy can be seated in holder 10. Fan intakes air through hole 111, the air then passes through air filer 112, along blades of fan 3 and then it goes toward reflector 222 of light source 233. Socket 224 of light source 223 holds air deflector 225 that directs the airflow in the area of reflector 222 and brings the air into annular gap 131 between inner casing 2 and front section 120 of outer casing 1. Air passes through this gap 131 along Brewster polariser 5 and along output section 230 of inner casing 2 into the first hole 121 of front section 120 of outer casing 1 and out of the instrument through the first hole 121 in the direction to the treated area along massage ring 240. Light rays generated by light source 223 impinge on mirror surface of reflector 223 from which the rays are directed in a bundle to the surface of glass plates 511 of Brewster polariser 5, from which the rays are reflected under the known Brewster angle and being linear-polarised, they pass through filter 61. It is an advantage, that lens 6 is located behind filter 61 in the direction of rays. This lens focuses rays into the focus located on the optical axis of lens 6 outside of inner casing 2. According to Figure 2, drawn glass plates 511 are connected into a system, in which they are close to each other. Glass plates 511 manufactured by means of the draw technology have irregularities on their surfaces. Thickness of these irregularities can vary, but according to experience, it is advantageous the thickness to be about 1 mm. Due to air humidity, the plates are not self-sticky. There is zero mutual distance at contact points of adjacent glass plates 511 and the distance can vary in the cavity areas. Glass plates 511 have cavities 512 of various sizes between them, for example only microscopic ones that enable a different refraction of light. When the bundle of rays impinges on the first drawn glass plate 511, considerable part of rays is reflected under the Brewster angle. Part of the rays pass through the drawn glass plate 511 and cavities 512 of various sizes and impinge on the second of glass plates 511 under a changed angle as a result of light refraction index and depending on the thickness of glass plate 511 and cavity 512. Part of these rays is reflected back under the angle of incidence and part of the rays pass again through the drawn glass plate 511. This physical phenomenon repeats on every next glass plate 511 and terminates on black insert 520 with profiles on surface 521 that is bound on the last drawn glass plate 511. Drawn glass plates 511 can also have a different thickness. The optimum polarisation effect is achieved by means of a suitable combination of drawn glass plates 511. This increases efficiency of the Brewster polariser while considerably decreasing production costs on manufacture of drawn glass plates 511. Black insert 520 is seated on bearing plate 510 inserted into the recess in the hole of inner casing 2. Thanks to profiles of surface 521, black insert 521 is flexible and thus suitably compensates production tolerances and thermal expansion of the system of assembled drawn glass plates 511 so that they are permanently in close contact with each other. The profiled surface 521 of black plate 520 also enables the product to be marked in a simple way. A suitable shape of the recess formed in the profiled surface 521 can be used to mark instructions for use or manufacturer's trademark etc., because the profiled surface 521 is visible during operation of the instrument when viewed in the direction against the outgoing polarised light. Figure 3 shows the front section 120 of outer casing 1, from which collar 233 of output section 230 not shown in the figure is protruding from the first section 121. According to Figure 4, the simple implementation of massage ring 240 is provided with flexible massage elements 241. According to Figure 5, permanent magnets 242 or electromagnets 243 connected with electrical socket 247 for connection to a power supply source are located in the body of massage ring 240. According to Figure 6, it is an advantage, that massage ring 240 is a movable component of vibrator 248. Vibrator 248 consists of collar 234 as a fixed component embedded in output section 230 of inner casing 2 and/or hole 121 of outer casing 1, further as an example, it consists of flexible attachment 246, massage ring 240 that forms the movable component and tools for generating oscillating motions of massage ring 240 that, as an example, consist of electromagnets 243 connected with electric terminal 247 for connection to electrical power supply. The shown flexible attachment 246 is only as an example and can also be replaced with push-type attachment of massage ring 240 in collar 234 for oscillating motions either in axial direction or for rotary-oscillating motions along the axis of massage ring 240. When implementing massage ring 240, together with light effects it is also possible to perform massage of surface of the treated area. Circling or oscillating motions of the instrument, when the instrument is manual-controlled utilise the advantage of flexible massage elements 241. It is an advantage, that the manual control can be substituted with a vibrator as mentioned above. It is an advantage, that programmable functions of controller 42 can be utilised, which enable programmable amplification and attenuation of vibrations, light flow, flow speed and temperature of the airflow. Exhausting of warm air onto the treated area is also important for the healing effect, namely because warm air according of the invention is exhausted outside the circle formed by massage ring 240. According to the advantageous implementation of the instrument for light therapy according to Figure 7, reflector 222 has a parabolic reflection plane 252. Another elliptic reflector 221 with elliptic reflection plane 251 is situated against the parabolic reflector 222 so that optical axes 501 and 502 are identical. Light source 223 is located in primary focus F1 of part of elliptic reflector 221 and whose secondary focus is identical with focus F2 of parabolic reflector 222, i.e. with the focus of parts of parabolic reflector 222, if the reflector is divided into several parts. Reflection plane 251 of elliptic reflector 221 is considerably smaller than reflection plane 252 of parabolic reflector 222. Such configuration considerably restricts diffusion of light radiated by light source 223. It is an advantage, that after passing through filter 61, the bundle of rays reflected from the Brewster polariser is focused by lens 6 into focus F3 located on optical axis 503 outside the outer casing that is not shown. The instrument for light therapy can be used not only for stimulation of biological processes when healing surface wounds on bodies, but also in other branches of medicine, cosmetics and biology. It can be used in physical treatment in the branch of mouth, jaw and face surgery.

## Claims

1. An instrument for light therapy, consisting of an outer casing (1) in with a front section terminating in a first hole (231), in said outer casing comprising an inner casing (2) with a light source (223), a reflector (222) and a Brewster polariser(s) consisting of a system of parallel glass plates (511) lying on each other, the output section of said inner casing reaching as far as to the first hole (231) of said outer casing and holding a light filter (6), said inner casing having a rear section that comprises a second hole (111), in which a fan (3) for the generation of a pressure difference between both holes is located,
**characterised in that** said
system of glass plates of said Brewster polariser (5) consists of drawn glass plates (511) with surface irregularities defining cavities (512) of unequal shape between said plates (511).

2. Instrument for light therapy according to claim 1,
further **characterised in that**
the outer side of said system of glass plates (511) is provided with an insert (520) having a black surface that has profile patterns on the side of said glass plates (511).

3. Instrument for light therapy according to claim 1,
further **characterised in that** a
massage ring (240) is allocated to said Brewster polariser, that is located in said output section (230) of the inner casing (2) and/or at the end of front part (120) of said outer casing (1).

4. Instrument for light therapy according to claim 3,
further **characterised in that** a
massage ring (240) is provided with flexible massage elements (241).

5. Instrument for light therapy according to claim 3,
further **characterised in that** a
massage ring (240) is provided with magnets (242, 243) for generating a magnetic field outside the output hole (231) of said inner casing (2).

6. Instrument for light therapy according to claim 5,
further **characterised in that** said magnets (242, 243) are permanent or with electrical excitation.

7. Instrument for light therapy according to claim 4,
further **characterised in that** said
massage ring (240) is a movable part of a vibrator (248) located at the output section (230) of the inner casing (2) and/or at the end of the front section (120) of the outer casing (1).

8. Instrument for light therapy according to claim 1 - 7,
further **characterised in that**
there is fan (3) allocated to said Brewster polariser (5), said fan being set for airflow direction from the second hole (111) of outer casing (1) toward its first hole (121).

9. Instrument for light therapy according to claim 1,
further **characterised by**
a lens (6) which is located in said output section (230) of the inner casing (2) to focus rays reflected from the system of glass plates (511) into a focus located outside the outer casing (2) on the optical axis of said lens (6).

## Patentansprüche

1. Lichttherapiegerät, bestehend aus einem äußeren Gehäuse (1), das einen vorderen Teil aufweist, der mit einer ersten Öffnung (231) endet, das genannte äußere Gehäuse umfassend ein Innengehäuse (2) mit einer Lichtquelle (233), einem Reflektor (222) und einem aus einem System paralleler, aufeinander liegender Glasschichten bestehenden Brewster-Polarisator (5), wobei der Austrittsteil des genannten Innengehäuses in die erste Öffnung des genannten Außengehäuses hineinreicht und einen Lichtfilter (6) enthält, das genannte Außengehäuse einen hinteren Teil mit zweiter Öffnung (111) aufweist, in der der Ventilator (3) zur Bildung einer Druckdifferenz zwischen beiden Öffnungen angebracht ist,
**gekennzeichnet dadurch, dass**
das genannte System der gläsernen Schichten des genannten Brewster-Polarisators (5) aus gezogenen Glasplatten (511) mit oberflächigen Unebenheiten gebildet ist, die zwischen den genannten Platten (511) Hohlräume (512) von ungleicher Form eingrenzen.

2. Lichttherapiegerät nach Anspruch 1,
**weiter gekennzeichnet dadurch, dass** das genannte Glassplattensystem (511) auf der äußeren Seite mit einer Einlage (520) versehen ist, die eine schwarze, auf der Seite der genannten Glasplatten (511) mit Profilmuster versehene Oberfläche hat.

3. Lichttherapiegerät nach Anspruch 1,
**weiter gekennzeichnet dadurch, dass** ein Massagering (240) dem Brewster-Polarisator (5) zugeordnet ist, der sich in dem genannten Ausgangsteil (230) des Innengehäuses (2) und/oder am Ende des Vorderteils (120) des genannten Außengehäuses (1) befindet.

4. Lichttherapiegerät nach Anspruch 3,
**weiter gekennzeichnet** t **dadurch**, dass der Massagering (240) mit elastischen Massageelementen (241) versehen ist.

5. Lichttherapiegerät nach Anspruch 3,
**weiter gekennzeichnet dadurch, dass** der Massagering (240) mit Magneten (242, 243) zum Aufbau eines Magnetfelds außerhalb der Austrittsöffnung (231) des Innengehäuses (2) versehen ist.

6. Lichttherapiegerät nach Anspruch 5,
**weiter gekennzeichnet dadurch, dass** die genannten Magnete (242, 243) permanent oder mit elektrischer Erregung sind.

7. Lichttherapiegerät nach Anspruch 4,
**weiter gekennzeichnet dadurch, dass** der Massagering (240) ein bewegliches Glied eines Vibrators (248) ist, der am Ausgangsteil (230) des Innengehäuses (2) und/oder am Ende des Vorderteils (120) des Außengehäuses (1) angeordnet ist.

8. Lichttherapiegerät nach einem der Ansprüche 1-7, **weiter gekennzeichnet dadurch, dass** dem genannten Brewster-Polarisator (5) ein Ventilator (3) zugeordnet ist, wobei der genannte Ventilator für die Luftströmungsrichtung ab der zweiten Öffnung (111) des Außengehäuses (1) zu dessen erster Öffnung (121) eingestellt ist.

9. Lichttherapiegerät nach Anspruch 1,
**weiter gekennzeichnet durch**
eine Linse (6), die im genannten Ausgangsteil (230) des Innengehäuses (2) dazu angeordnet ist, um die von dem Glasplattensystem (511) in den außerhalb des Innengehäuses (2) auf der optischen Achse der genannten Linse (6) liegenden Brennpunkt reflektierten Strahlen zu zentralisieren.

## Revendications

1. Un apareil pour la thérapie lumineuse, composé d'un étui (1) extérieur, avec la partie antérieure terminant par le premier orifice (231), l'étui extérieur mentionné comportant l'étui intérieur (2) avec une source lumineuse (223), avec un réflecteur (222) et avec un polarisateur (5) de Brewster formé d'un système des niveaux de verre (511) parallèles situés l'un sur l'autre, la partie de sortie de l'étui intérieur atteinant le premier orifice (231) de l'étui extérieur et comportant un filtre (6) lumineux, la partie postérieure de l'étui intérieur mentionné ayant le deuxième orifice (111), dans lequel un ventilateur (3) pour la formation de la différence de pression entre les deux orifices est situé
**caracterisé en ce que**
le système des niveaux de verre du polarisateur de Brewster (5) mentionné est formé des tableaux (511) de verre traits avec des inégalités de surface déterminantes entre les tableaux mentionnés (511) des creux (512) de la forme différente.

2. Un apareil pour la thérapie lumineuse selon la revendication 1
**caracterisé en ce**
**que** le système des niveaux de verre (511) est du coté extérieur muni d'une fourrure (520) avec la surface noire garnie sur le coté vers les tableaux de verre (511) d'un motif profilé.

3. Un apareil pour la thérapie lumineuse selon la revendication 1
**caracterisé en ce**
que'un anneau de massage (240) est aligné au polarisateur (5) de Brewster, arrangée dans la partie de sortie (230) de l'étui intérieur (2) mentionnée et/ou au bout de la partie antérieure (120) de l'étui extérieur (1) mentionné.

4. Un apareil pour la thérapie lumineuse selon la revendication 3
**caracterisé en ce**
**que** l'anneau de massage (240) est muni des éléments de massage flexibles (241).

5. Un apareil pour la thérapie lumineuse selon la revendication 3
**caracterisé en ce**
**que** l'anneau de massage (240) est muni des aimants (242, 243) pour la formation du champ magnétique à l'extérieur de l'orifice de sortie (231) de l'étui intérieur (2).

6. Un apareil pour la thérapie lumineuse selon la revendication 5
**caracterisé en ce**
**que** les aimants mentionnés (242, 243) sont permanents ou avec l'excitation électrique.

7. Un apareil pour la thérapie lumineuse selon la revendication 4
**caracterisé en ce que**
que l'anneau de massage (240) est la pièce mobile du vibrateur (248) arrangé à la partie de sortie (230) de l'étui intérieur (2) et/ou au bout de la partie antérieure (120) de l'étui extérieur (1)

8. Un apareil pour la thérapie lumineuse selon l'une quelconque revendication 1 - 7
**caractérisé en ce**
**qu'**un ventilateur (3) est aligné au polarisateur (5) de Brewster mentionné, le ventilateur mentionné est positionné à la direction de la circulation de l'air du deuxième orifice (111) de l'étui extérieur (1) au premier orifice (121) de celui-ci.

9. Un apareil pour la thérapie lumineuse selon la revendication 1
**caracterisé par**
une lentille (6) arrangée dans la partie de sortie (230) de l'étui intérieur (2) pour concentrer les rayons réfléchis du système des tableaux de verre (511) vers le foyer situé à l'extérieur de l'étui intérieur (2) sur l'axe optique de la lentille (6) mentionnée.
